# EUROPÄISCHE PATENTSCHRIFT

(11) **EP 0 484 765 B1**
(45) Veröffentlichungstag und Bekanntmachung des Hinweises auf die Patenterteilung: **26.06.1996**
(21) Anmeldenummer: 91118224.4
(22) Anmeldetag: 25.10.1991
(51) Int. Cl.: G01N 33/543, G01N 33/569

(54) **Verfahren zur Bestimmung von Proteinen in Körperflüssigkeiten und Mittel zur Durchführung des Verfahrens**
Method and means for determining proteins in body-fluids
Méthode et moyens de dosage de protéines dans des fluides corporels

(30) Priorität: 06.11.1990 DE 4035174
(43) Veröffentlichungstag der Anmeldung: 13.05.1992
(73) Patentinhaber: BIOTEST AG, D-63303 Dreieich (DE)
(72) Erfinder: Horn, Jürgen, Dr. Dipl.-Chem., W-6073 Egelsbach (DE); Nebel-Schickel, Heike, Dr. Dipl.-Biol., W-6451 Hammersbach-Marköbel (DE)
(74) Vertreter: Beil, Hans Chr., Dr.

(56) Entgegenhaltungen:
- EP-A- 0 133 272
- EP-A- 0 202 093
- EP-A- 0 303 062
- EP-A- 0 303 980
- EP-A- 0 445 650
- WO-A-90/10232
- US-A- 4 839 298
- US-A- 4 931 385

## Beschreibung

Die vorliegende Erfindung betrifft ein Verfahren zur Bestimmung von Proteinen in Körperflüssigkeiten nach dem Prinzip der Festphasenimmunreaktion und Mittel zur Durchführung desselben gemäß den Ansprüchen, wobei verbesserte Ergebnisse erzielt werden durch Vermeidung falsch positiver Reaktionen. Insbesondere eignet sich das erfindungsgemäße Verfahren zum Nachweis von Antikörpern gegen HIV oder des Cytomegalievirus in Serum oder Plasma.

Die Bestimmung von Proteinen, wie Antigenen, Antikörpern oder Haptenen in Körperflüssigkeiten, wie z.B Seren oder Plasma, stellt ein hochempfindliches Verfahren dar. Dabei werden die Bestimmungen unter Verwendung einer festen Phase in der sogenannten "Sandwich"-Technik oder Modifikationen hiervon durchgeführt. Hierzu wird ein Reaktionspartner an die Festphase, wie Polystyrolkugeln, Polystyrollatex, oder an die Oberfläche von Mikrotestplatten gebunden.
Die Festphase kann darüberhinaus auch aus anderen, dem Fachmann bekannten Polymeren bestehen und direkt mit einem Reaktionspartner, z.B. dem Antigen, beschichtet werden. Anschließend wird diese mit dem in der Körperflüssigkeit vorhandenen zu bestimmenden Protein zur Reaktion gebracht und auf übliche Weise nachgewiesen, wie z.B. mittels Enzymmarkierungen (vgl. DE-OS 39 30 376.4), über radioaktive Markierung oder mittels Chelatkomplexen der Seltenen Erden.

In der EP-A 0 292 809 wird ein Verfahren zur Bestimmung von Proteinen offenbart, in welchem ein Partner in einer wäßrigen Lösung mit dem an eine Festphase kovalent gebundenen Partner reagiert, wobei der Puffer ein nichtionisches Tensid der Formel

CH₃(CH₂)ₘ-R-(CH₂-CH₂-O)ₙ-H

mit
R = O, NH, CH=CH-(CH₂)ₚ-O
m = 3 bis 26
n = 7 bis 40 und
p = 5-15
enthält.

Ein derartiger Puffer soll allgemein schwankende bzw. zu niedrige oder zu hohe Ergebnisse in immunologischen Tests verhindern. Jedoch wird hier kein Hinweis darauf gegeben, insbesondere beim Nachweis bestimmter Proteine, wie z.B. Viren, die dabei oftmals auftretenden falsch positiven Ergebnisse zu unterdrücken.

Dies wird jedoch vor allem bei der Bestimmung von HIV- oder Cytomegalievirus (CMV)-Antikörpern beobachtet, ohne daß tatsächlich positive Proben vorliegen. Vor allem gepoolte Seren oder Plasmen können beim Antikörpertest gegen HIV oder CMV unspezifisch positiv reagieren.

Die EP-A 0 303 062 beschreibt ein stabilisiertes Peroxidasepräparat für Enzymimmuntests, wobei als Stabilisatoren Hühneralbumin oder Kalbsserum zusammen mit Polyvinylpyrrolidon und/oder Calciumpropionat eingesetzt werden.

Die US-A 4 931 385 offenbart eine lyophilisierte Zusammensetzung zur Anwendung in Enzymimmuntests, welche eine die Antigenbindung verstärkende Substanz, wie Polyvinylpyrrolidon, ein Tensid sowie eine Zuckerverbindung enthält.

Die EP-A 0 303 980 betrifft die Verwendung von Serumproteinen und analogen Proteinen als Blocksubstanzen, um freie Oberflächen des Trägermaterials bei Immunoassays abzusättigen.

Die EP-A 0 133 272 offenbart Verdünnungspuffer für Enzymimmunoassays, wobei dem Inkubationsmilieu ein Detergens zugessetzt wird.

Die EP-A 0 445 650 mit der Priorität vom 5. März 1990 und veröffentlicht am 11. September 1991 mit Benennung der Vertragsstaaten DE, ES, FR, IT betrifft ein Verfahren zur Bestimmung von Anti-HIV-Antikörpern in Urinproben, denen ein Verdünnungspuffer zugsetzt wird, der neben herkömmlichen Puffersubstanzen wie Phosphat, TRIS, HEPES, Salze wie NaCl oder Natriumazid, Schaf- und Kalbsserum, Tenside, Chelatbildner und weitere für Urinproben spezifische Zusätze enthält.

In der US-PS 4 839 298 wird hierzu ein Verfahren offenbart zur Inaktivierung von HIV Antikörpern mit einer dadurch bedingten höheren Spezifität des Immunoassays. Hierzu wird als Inaktivierungspuffer ein Salz/Phosphatpuffer zusammen mit 0,05 bis 1% Salz, vorzugsweise Chloridsalz, und einem nichtionischen Tensid (0,05 bis 0,5 %) eingesetzt, so daß der Inaktivierungspuffer eine bestimmte Leitfähigkeit (21 bis 35 mS/cm) aufweist.

Zwar ist mit einem solchen System eine Inaktivierung von HIV möglich, jedoch wird hier nicht beschrieben, wie HIV-Antikörper reproduzierbar und definitiv bei sicherer Unterdrückung falscher Ergebnisse bestimmt werden können.

Aufgabe vorliegender Erfindung ist daher, ein Verfahren zur Bestimmung von Proteinen in Körperflüssigkeiten, insbesondere von HIV- oder CMV-Antikörpern in Seren oder Plasmen, bereitzustellen, durch das zuverlässige Meßergebnisse erzielt werden und unspezifisch positive Reaktionen unterdrückt werden können.

Diese Aufgabe wird erfindungsgemäß dadurch gelöst, daß man der Probe, die das zu bestimmende Protein enthält, einen Verdünnungspuffer zusetzt, der neben einem Phosphat- oder Carbonatpuffer zusätzlich noch 5 bis 75 Vol.-% tierische Seren und/oder 0,01 bis 5 Gew. bzw. Vol.-% eines nichtionischen Tensids sowie 0,02-0,8 M anorganische Salze, ausgewählt aus Natriumfluorid, Natriumbromid oder Natriumiodid, und/oder 0,02-0,8M Natriumpropionat enthält.

Zu den erfindungsgemäß zu verwendenden tierischen Seren gehören insbesondere Kalbs- und Ziegenserum, die vor ihrer Verwendung inaktiviert (beispielsweise bei 55°C, 1 Stunde) und danach sterilfiltiert werden. Auch Gemische dieser Seren eignen sich für die erfindungsgemäßen Zusammensetzungen, wie etwa solche aus 25% Kalbs- und 25% Ziegenserum.

Als nichtionische Tenside eignen sich u.a. veresterte Polyoxyethylene z.B. der Formel

CH₃-(CH₂)ₘ-R-(CH₂-CH₂-O)ₙ-H

mit
R = O, NH, CH=CH-(CH₂)ₚ-O,
m = 6-8,
n = >40,
p = 6-8
(Polymerisationsgrad >40)
und veretherte Polyoxyethylene.

Insbesondere bevorzugt werden Tween 20^{(R)}, Tergitol^{(R)}, Triton^{(R)} und Lubrol^{(R)}. Dabei werden sie im Bereich von 0,01 bis 5% eingesetzt, bezogen auf das Gewicht bei festen Stoffen bzw. auf Volumenanteile bei flüssigen Produkten.

Unter den genannten anorganischen Salzen ist Natriumfluorid besonders bevorzugt ist.

Mit den so gekennzeichneten Verdünnungspuffern ist es möglich, zuverlässig und reproduzierbar Proteine in Körperflüssigkeiten zu bestimmen.
Dabei haben sich folgende Zusammensetzungen als besonders geeignet erwiesen und sind daher bevorzugt:
- Verdünnungspuffer, enthaltend Phosphat-gepufferte Kochsalzlösung mit 0,05 Vol.-% Tween® 20, 5-20 Vol.-% Kalbsserum und anorganische Salze (0,02 bis 0,8 M), insbesondere Natriumfluorid (vorzugsweise 0,1-0,5 M) und bzw. oder Natriumpropionat (0,1 bis 0,5 M).
- Verdünnungspuffer, enthaltend Phosphat-gepufferte Kochsalzlösung mit 5-25 %, insbesondere 5-20 Vol.-% und ganz besonders bevorzugt 10 Vol.-% Kalbsserum, zusammen mit Natriumfluorid (0,02 bis 0,8 M, insbesondere 0,1 bis 0,5 M) und Natriumpropionat (0,02 bis 0,8 M).

Eine weitere besonders geeignete Gruppe von erfindungsgemäß verwendbaren Verdünnungspuffern sind solche, enthaltend einen Carbonatpuffer (wie z.B. 0,05 M) mit Kalbsserum, insbesondere 5 bis 25 Vol.-%, und/oder Tween® 20 (0,1-0,5 Vol.-%) und zusätzlich anorganische Salze wie oben beschrieben, insbesondere Natriumfluorid (0,02 bis 0,8 M) aufweist. Weiterhin besonders geeignet sind Zusammensetzungen der obigen Art, die zusätzlich zu Natriumfluorid bzw. anstelle desselben Natriumpropionat (insbesondere 0,12 bis 0,16 M) enthalten.

Mit den so gekennzeichneten Puffern wird die zu bestimmende Probe versetzt und nach der Festphasenmethode, wie in der DE-OS 39 30 376.4 beschrieben, bestimmt. Ein Reaktionspartner ist dabei an die Festphase (Polystyrol, Mikrotestplatte u.a. Polymere) gebunden und wird mit dem in der Probe vorliegenden Reaktionspartner umgesetzt. Der Nachweis erfolgt mittels eines dritten markierten Partners, der an den weiten bindet. Die Markierung kann z.B. ein Enzym (Peroxidase, alkalische Phosphatase) oder radioaktiv (¹²⁵J) oder über Chelate der Seltenen Erden (Europiumchelate) mit nach folgendem Fluoreszenznachweis erfolgen.

Die nachfolgenden Beispiele erläutern die vorliegende Erfindung.

### Beispiel 1

7 verschiedene europäische Seren der nachstehenden Klassifikation wurden nach der Sandwich-Technik auf HIV-Antikörper untersucht. Der Nachweis erfolgte dabei enzymatisch durch Messung der optischen Extinktion des bei der Nachweisreaktion entstandenen Produkts mit einem Spektralphotometer (492 nm, Referenzwellenlänge 570-650 nm), vgl. auch Testvorschrift "Biotest-Anti-HIV-1/-2 recombinant", Zul.-Nr.: 105a/90 bzw. Biotest Anti-CMV IgG ELISA", Zul.-Nr. 62a /86 gemäß Paul-Ehrlich-Institut.

Die Molaritätsangaben beziehen sich auf die Molarität der entsprechenden Substanz im fertigen Verdünnungspuffer.

### Serenklassifikation:

Serum 1 = negatives europäisches Serum
Serum 2 = positives europäisches Serum
Serum 3 = Serum 2 in 1:100 Verdünnung
Serum 4 = Serum 2 in 1:200 Verdünnung
Serum 5 = negatives europäisches Serum
Serum 6 = negativer europäischer Serumpool
Serum 7 = negativer europäischer Serumpool

Die Ergebnisse dieser Untersuchungen sind in nachfolgender Tabelle 1 zusammengestellt:

**Tabelle 1**

| Verdünnungspuffer | Serumprobe | | | | | | |
|---|---|---|---|---|---|---|---|
| | 1 | 2 | 3 | 4 | 5 | 6 | 7 |
| A = Phosphat gepufferte Kochsalzlösung mit 0,05 Vol% Tween 20® | 0,022 | >3,0 | 1,167 | 0,546 | 0,410 | 0,171 | 0,368 (Vgl) |
| B = A + 0,1 Molar NaF | 0,001 | >3,0 | 0,748 | 0,397 | 0.019 | 0.007 | 0.054 (Erf) |
| C = A + 0,5 Molar NaF | 0,002 | >3,0 | 0,488 | 0,316 | 0,018 | 0,004 | 0,018 (Erf) |
| D = A + 0,1 Molar Propionsäure | -0,001* | >3,0 | 0,568 | 0,334 | 0,020 | 0,003 | 0,028 (Erf) |
| E = A + 0,1 Molar Propionsäure Natriumsalz + 0,1 Molar NaF | -0,004* | >3,0 | 0,827 | 0,435 | 0,012 | 0,002 | 0,016 (Erf) |
| F = A + 0,1 Molar Natriumjodid | 0,001 | >3,0 | 0,815 | 0,446 | 0,025 | 0,015 | 0,014 (Erf) |
| G = A + 0,1 Molar Natriumbromid | 0,002 | >3,0 | 0,832 | 0,458 | 0,022 | 0,012 | 0,016 (Erf) |
| H = A+10Vol% Kalbsserum +0,5Vol% NP40 *** +0,05 Molar NaJ +0,1 Molar Propionsäure Natriumsalz | 0,000 | >3,0 | 1,059 | 0,628 | 0,012 | 0,002 | 0,004 (Erf) |
| J = A + 25Vol% Kalbsserum +0,15 Molar Natriumflorid +0,1 Molar Propionsäure Natriumsalz | -0,001* | >3,0 | 1,156 | 0,657 | 0,010 | 0,001 | 0,002 (Erf) |

| | | | | | | | |
|---|---|---|---|---|---|---|---|
| * Durch Abzug des Reagenzienleerwertes können sich bei negativen Proben negative Extinktionswerte ergeben. ** POES 100 = Polyoxyethylenstearat 100 (Polymerisationsgrad des Oxyethylens = 100). | | | | | | | |
| *** Octylphenol-ethylenoxid-Kondensat Noridet P40 | | | | | | | |

Extinktionswerte <0,3 entsprechen einem zuverlässigen (zutreffenden) negativen Ergebnis, Werte >0,3 entsprechen einem definitiven positiven Ergebnis.

Wie dieser Versuchsreihe entnommen werden kann, werden die Proben mit einem herkömmlichen Verdünnungspuffer aus Phosphat gepufferter Kochsalzlösung mit Tween 20® nicht richtig erkannt. Die Proben 5 und 7 zeigen positive Ergebnisse, obgleich es sich hierbei um HIV-negative Seren handelt.

Die mit einem Verdünnungspuffer nach dem erfindungsgemäßen Verfahren durchgeführten Bestimmungen zeigen sämtlich richtige Werte, also keine falsch positiven Ergebnisse, selbst bei einem Verdünnungsgrad der Probe von 1:100 oder sogar 1:200.

### Beispiel 2

Analog wie in Beispiel 1 wurden negative afrikanische Seren 8-12 durch Messung der Extinktionswerte auf HIV-Antikörperanwesenheit bestimmt.

Die Ergebnisse sind in Tabelle 2 zusammengefaßt:

**Tabelle 2**

| Verdünnungspuffer | 8 | 9 | 10 | 11 | 12 |
|---|---|---|---|---|---|
| A | 1,486 | 0,488 | 0,712 | 0,255 | 0,981(Vgl) |
| Q** | 0,227 | 0,084 | 0,092 | 0,018 | 0,112(Erf) |
| B | -* | 0,097 | 0,098 | 0,155 | 0,199(Erf) |

| | | | | | |
|---|---|---|---|---|---|
| * Keine Messung durchgeführt | | | | | |
| ** Zusammensetzung Puffer Q: A + 25% Kalbsserum + 0,5% NP40 + 0.05 M NaJ + 0,1 M Natriumpropionat | | | | | |

Wie diese Ergebnisse zeigen, können mit Hilfe des erfindungsgemäßen Verfahrens auch afrikanisch Seren zuverlässig bestimmt werden, obwohl, wie auch der Vergleich mit einem herkömmlichen Standardpuffer A zeigt, insbesondere bei afrikanischen Seren oftmals falsch positive Ergebnisse erzielt werden.

### Beispiel 3

Wie in Beispiel 1 und 2 wurden nachfolgend europäische Seren 7 und 13-16 der untenstehenden Spezifikation auf CMV-Antikörper hin untersucht.

### Getestetes Serum:

13 = positives Serum
14 = positives Serum 1:100 in negativem Serum verdünnt
15 = negatives Serum
16 = negatives Serum

Die Ergebnisse sind in Tabelle 3 zusammengestellt:

**Tabelle 3**

| Verdünnungspuffer | Serum | | | | |
|---|---|---|---|---|---|
| | 7 | 13 pos. | 14 pos. | 15 neg. | 16 neg. |
| A | 0,168 | 2,417 | 0,228 | 0,256 | 0,306 |
| Q | 0,014 | 2,428 | - | 0,049 | 0,026 |

### Beispiel 4

Wie in Beispiel 1 wurden nachfolgende Zusammensetzungen im HIV-Test auf Anwesenheit von HIV-Antikörpern hin untersucht. Dabei wurden die in Beispiel 2 verwendeten afrikanischen negativen Seren 8-12 sowie Serum 4 gemäß Beispiel 2 (positives europäisches Serum 1:200 verdünnt) eingesetzt.

Die Ergebnisse sind in nachfolgender Tabelle 4 zusammengefaßt:

**Tabelle 4**

| Verdünnungspuffer | Serumprobe | | | | | |
|---|---|---|---|---|---|---|
| | 8 | 9 | 10 | 11 | 12 | 4 |
| A+25Vol.-% Kalbsserum +0,15 Molar Natriumfluorid +0,1 Molar Propionsäure-Natriumsalz | 0,121 | 0,072 | 0,098 | 0,002 | 0,117 | 0,699 |

Wie diese Ergebnisse zeigen, können mit den erfindungsgemäßen Puffern, insbesondere jene enthaltend Serum und/oder Tensid, Natriumfluorid und Natriumpropionsäure, HIV-Antikörper zuverlässig bestimmt bzw. ihre Anwesenheit zuverlässig ausgeschlossen werden.

### Beispiel 5

Nachfolgend wurden die afrikanischen Seren 8-12 gemäß Beispiel 2 sowie ein positives europäisches Serum 14, 1:100 in negativem Serum verdünnt, gemäß Beispiel 3 auf HIV-Antikörper nach der beschriebenen Methode untersucht.

Die Ergebnisse sind in nachfolgender Tabelle 5 zusammengestellt:

**Tabelle 5**

| Verdünnungspuffer für die Proben | Extinktionswerte der Proben | | | | | |
|---|---|---|---|---|---|---|
| | 8 | 9 | 10 | 11 | 12 | 24 |
| 0,05 Molar Carbonatpuffer | 1,655 | 0,617 | 0,938 | 0,327 | 1,103 | 0,652 (Vgl) |
| 0,05 Molar Carbonatpuffer +25 Vol.-% Kalbsserum +0,15 Molar Natriumfluorid | 0,135 | 0,056 | 0,092 | 0,018 | 0,114 | 0,662 (Erf) |
| 0,05 Molar Carbonatpuffer +25 Vol.-% Kalbsserum +0,15 Molar Propionsäure-Natriumsalz | 0,149 | 0,037 | 0,067 | 0,022 | 0,098 | 0,675 (Erf) |

Diese Werte belegen, daß auch die erfindungsgemäßen Carbonatpuffer mit den genannten Zusätzen sicher und reproduzierbar bestimmt bzw. ausgeschlossen werden können.

## Patentansprüche (Patentansprüche für folgende(n) Vertragsstaat(en): AT, BE, CH, LI, DE, FR, GB, IT, NL, SE)

1. Verfahren zur Vermeidung falsch positiver Reaktionen bei der Bestimmung von Proteinen in Körperflüssigkeiten, wobei man einen Reaktionspartner an eine feste Phase bindet und mit dem zu bestimmenden Partner aus der Körperflüssigkeit zur Reaktion bringt, wobei der Nachweis enzymatisch, radioaktiv oder mittels Fluoreszenzanalyse erfolgt, dadurch gekennzeichnet, daß man der die zu bestimmende Probe enthaltenden Lösung einen Verdünnungspuffer zusetzt, enthaltend 5-75% eines tierischen Serums und/oder 0,01 bis 5 Gew.-% bzw. Vol.-% eines nichtionischen Tensids sowie 0,02 bis 0,8 M anorganische Salze, ausgewählt aus Natriumfluorid, Natriumiodid oder Natriumbromid und/oder 0,02 bis 0,8 M Natriumpropionat in einem Phosphat- oder Carbonatpuffer.

2. Verfahren nach Anspruch 1, dadurch gekennzeichnet, daß man einen Verdünnungspuffer verwendet, der Phosphat gepufferte Kochsalzlösung mit 0,05 Vol.-% Tween® 20, 5-20 Vol.-% Kalbsserum und 0,02-0,8 M Natriumfluorid enthält.

3. Verfahren nach Anspruch 1, dadurch gekennzeichnet, daß man einen Verdünnungspuffer verwendet, der 5-20 Vol.-% Kalbsserum und Phosphat-gepufferte Kochsalzlösung und 0,02 bis 0,8 M Natriumfluorid enthält.

4. Verfahren nach Anspruch 1, dadurch gekennzeichnet, daß man einen Verdünnungspuffer verwendet, der Phosphatgepufferte Kochsalzlösung, 5-20 Vol.-% Kalbsserum und/oder 0,05 Vol.-% Tween® 20 sowie 0,02 bis 0,8 M Natriumpropionat enthält.

5. Verfahren nach einem der Ansprüche 2 oder 3, dadurch gekennzeichnet, daß der eingesetzte Verdünnungspuffer zusätzlich 0,02 bis 0,8M Natriumpropionat enthält.

6. Verfahren nach Anspruch 1, dadurch gekennzeichnet, daß man einen Verdünnungspuffer verwendet, der einen Carbonatpuffer enthält.

7. Verfahren nach Anspruch 6, dadurch gekennzeichnet, daß man einen Verdünnungspuffer verwendet, der 0,05 M an Carbonatpuffer ist und 5-25 Vol-% Kalbsserum und 0,02 bis 0,8 M Natriumfluorid enthält.

8. Verfahren nach Anspruch 1, dadurch gekennzeichnet, daß man einen Verdünnungspuffer verwendet, der 0,05 M an Carbonatpuffer ist und 5-25 Vol.-% Kalbsserum und 0,02 bis 0,8 M Natriumpropionat enthält.

9. Verfahren nach einem der Ansprüche 1 bis 8, dadurch gekennzeichnet, daß die zu bestimmenden Proteine in der Körperflüssigkeit Antikörper gegen HIV in Serum oder Plasma sind.

10. Verfahren nach einem der Ansprüche 1 bis 8, dadurch gekennzeichnet, daß die zu bestimmenden Proteine in der Körperflüssigkeit Antikörper gegen das Cytomegalievirus in Serum oder Plasma sind.

11. Verdünnungspuffer zur Vermeidung falsch positiver Reaktion bei der Bestimmung von Proteinen in Körperflüssigkeiten, enthaltend 5-75% eines tierischen Serums und/oder 0,01 bis 5 Gew.-% bzw. Vol.-% eines nichtionischen Tensids sowie 0,02 bis 0,8 M anorganische Salze, ausgewählt aus Natriumfluorid, Natriumiodid oder Natriumbromid und/oder 0,02 bis 0,8 M Natriumpropionatin einem Phosphat- oder Carbonatpuffer.

12. Verdünnungspuffer gemäß Anspruch 11, dadurch gekennzeichnet, daß er Phosphat-gepufferte Kochsalzlösung mit 0,05 Vol.-% Tween® 20 und 5 bis 20 Vol.-% Kalbsserum und 0,02 bis 0,8 M anorganische Salze, ausgewählt aus Natriumfluorid, Natriumiodid oder Natriumbromid enthält.

13. Verdünnungspuffer nach Anspruch 12, dadurch gekennzeichnet, daß er zusätzlich 0,02 bis 0,8 M Natriumpropionat enthält.

14. Verdünnungspuffer nach Anspruch 11, dadurch gekennzeichnet, daß er einen Carbonatpuffer, 5 bis 25 Vol.-% Kalbsserum und 0,02 bis 0,8 M anorganische Salze, ausgewählt aus Natriumfluorid, Natriummiodid oder Natriumbromid enthält.

15. Verdünnungspuffer nach Anspruch 14, dadurch gekennzeichnet, daß er zusätzlich 0,02 bis 0,8 M Natriumpropionat enthält.

## Patentansprüche (Patentansprüche für folgende(n) Vertragsstaat(en): ES)

1. Verfahren zur Vermeidung falsch positiver Reaktionen bei der Bestimmung von Proteinen in Körperflüssigkeiten, wobei man einen Reaktionspartner an eine feste Phase bindet und mit dem zu bestimmenden Partner aus der Körperflüssigkeit zur Reaktion bringt, wobei der Nachweis enzymatisch, radioaktiv oder mittels Fluoreszenzanalyse erfolgt, dadurch gekennzeichnet, daß man der die zu bestimmende Probe enthaltenden Lösung einen Verdünnungspuffer zusetzt, enthaltend 5-75% eines tierischen Serums und/oder 0,01 bis 5 Gew.-% bzw. Vol.-% eines nichtionischen Tensids sowie 0,02 bis 0,8 M anorganische Salze, ausgewählt aus Natriumfluorid, Natriumiodid oder Natriumbromid und/oder 0,02 bis 0,8 M Natriumpropionat in einem Phosphat- oder Carbonatpuffer.

2. Verfahren nach Anspruch 1, dadurch gekennzeichnet, daß man einen Verdünnungspuffer verwendet, der Phosphat-gepufferte Kochsalzlösung mit 0,05 Vol.-% Tween® 20, 5-20 Vol.-% Kalbsserum und 0,02-0,8 M Natriumfluorid enthält.

3. Verfahren nach Anspruch 1, dadurch gekennzeichnet, daß man einen Verdünnungspuffer verwendet, der 5-20 Vol.-% Kalbsserum und Phosphat-gepufferte Kochsalzlösung und 0,02 bis 0,8 M Natriumfluorid enthält.

4. Verfahren nach Anspruch 1, dadurch gekennzeichnet, daß man einen Verdünnungspuffer verwendet der Phosphat-gepufferte Kochsalzlösung, 5-20 Vol.-% Kalbsserum und/oder 0,05 Vol.-% Tween® 20 sowie 0,02 bis 0,8 M Natriumpropionat enthält.

5. Verfahren nach einem der Ansprüche 2 der 3, dadurch gekennzeichnet, daß der eingesetzte Verdünnungspuffer zusätzlich 0,02 bis 0,8M Natriumpropionat enthält.

6. Verfahren nach Anspruch 1, dadurch gekennzeichnet, daß man einen Verdünnungspuffer verwendet, der einen Carbonatpuffer enthält.

7. Verfahren nach Anspruch 6, dadurch gekennzeichnet, daß man einen Verdünnungspuffer verwendet, der 0,05 M an Carbonatpuffer ist und 5-25 Vol-% Kalbsserum und 0,02 bis 0,8 M Natriumfluorid enthält.

8. Verfahren nach Anspruch 1, dadurch gekennzeichnet, daß man einen Verdünnungspuffer verwendet, der 0,05 M an Carbonatpuffer ist und 5-25 Vol.-% Kalbsserum und 0,02 bis 0,8 M Natriumpropionat enthält.

9. Verfahren nach einem der Ansprüche 1 bis 8, dadurch gekennzeichnet, daß die zu bestimmenden Proteine in der Körperflüssigkeit Antikörper gegen HIV in Serum oder Plasma sind.

10. Verfahren nach einem der Ansprüche 1 bis 8, dadurch gekennzeichnet, daß die zu bestimmenden Proteine in der Körperflüssigkeit Antikörper gegen das Cytomegalievirus in Serum oder Plasma sind.

## Claims (Claims for the following Contracting State(s): AT, BE, CH, LI, DE, FR, GB, IT, NL, SE)

1. Process for avoiding false positive reactions in the determination of proteins in body fluids, wherein one reactant is bound to a solid phase and reacted with the reactant to be determined from the body fluid, wherein detection is achieved enzymatically, radioactively or by fluorescence analysis, characterised in that a dilution buffer is added to the solution containing the specimen to be determined, which buffer contains 5-75% of an animal serum and/or 0.01 to 5 wt.% or vol.% of a non-ionic surfactant together with 0.02 to 0.8 M of inorganic salts selected from among sodium fluoride, sodium iodide or sodium bromide and/or 0.02 to 0.8 of sodium propionate in a phosphate or carbonate buffer.

2. Process according to claim 1, characterised in that a dilution buffer is used which contains a phosphate-buffered common salt solution with 0.05 vol.% of Tween® 20, 5-20 vol.% of calf serum and 0.02 to 0.8 M of sodium fluoride.

3. Process according to claim 1, characterised in that a dilution buffer is used which contains 5-20 vol.% of calf serum and phosphate-buffered common salt solution and 0.02 to 0.8 M of sodium fluoride.

4. Process according to claim 1, characterised in that a dilution buffer is used which contains phosphate-buffered common salt solution, 5-2 vol.% of calf serum and/or 0.05 vol.% of Tween® 20 together with 0.02 to 0.8 M of sodium propionate

5. Process according to one of claims 2 or 3, characterised in that the dilution buffer used additionally contains 0.02 to 0.8M of sodium propionate.

6. Process according to claim 1, characterised in that a dilution buffer is used which contains a carbonate buffer.

7. Process according to claim 6, characterised in that a dilution buffer is used which has carbonate buffer concentration of 0.05 M and contains 5-25 vol.% of calf serum and 0.02 to 0.8 M of sodium fluoride.

8. Process according to claim 1, characterised in that a dilution buffer is used which has a carbonate buffer concentration of 0.05 M and contains 5-25 vol.% of calf serum and 0.02 to 0.8 M of sodium propionate.

9. Process according to one of claims 1 to 8, characterised in that the proteins to be determined in the body fluid are antibodies to HIV in serum or plasma.

10. Process according to one of claims 1 to 8, characterised in that the proteins to be determined in the body fluid are antibodies to the cytomegalovirus in serum or plasma.

11. Dilution buffer to avoid false positive reactions in the determination of proteins in body fluids containing 5-75% of an animal serum and/or 0.01 to 5 wt.% or vol.% of a non-ionic surfactant together with 0.02 to 0.8 M of inorganic salts selected from among sodium fluoride, sodium iodine or sodium bromide and/or 0.02 to 0.8 M of sodium propionate in a phosphate or carbonate buffer.

12. Dilution buffer according to claim 11, characterised in that it contains phosphate-buffered common salt solution with 0.05 vol.% of Tween® 20 and 5 to 20 vol.% of calf serum and 0.02 to 0.8 M of inorganic salts selected from among sodium fluoride, sodium iodide or sodium bromide.

13. Dilution buffer according to claim 12, characterised in that it additionally contains 0.02 to 0.8 M of sodium propionate.

14. Dilution buffer according to claim 11, characterised in that it contains a carbonate buffer, 5 to 25 vol.% of calf serum and 0.02 to 0.8 M of inorganic salts selected from among sodium fluoride, sodium iodide or sodium bromide.

15. Dilution buffer according to claim 14, characterised in that it additionally contains 0.02 to 0.8 M of sodium propionate.

## Claims (Claims for the following Contracting State(s): ES)

1. Process for avoiding false positive reactions in the determination of proteins in body fluids, wherein one reactant is bound to a solid phase and reacted with the reactant to be determined from the body fluid, wherein detection is achieved enzymatically, radioactively or by fluorescence analysis, characterised in that a dilution buffer is added to the solution containing the specimen to be determined, which buffer contains 5-75% of an animal serum and/or 0.01 to 5 wt.% or vol.% of a non-ionic surfactant together with 0.02 to 0.8 M of inorganic salts selected from among sodium fluoride, sodium iodide or sodium bromide and/or 0.02 to 0.8 M of sodium propionate in a phosphate or carbonate buffer.

2. Process according to claim 1, characterised in that a dilution buffer is used which contains a phosphate-buffered common salt solution with 0.05 vol.% of Tween® 20, 5-20 vol.% of calf serum and 0.02 to 0.8 M of sodium fluoride.

3. Process according to claim 1, characterised in that a dilution buffer is used which contains 5-20 vol.% of calf serum and phosphate-buffered common salt solution and 0.02 to 0.8 M of sodium fluoride.

4. Process according to claim 1, characterised in that a dilution buffer is used which contains phosphate-buffered common salt solution, 5-20 vol.% of calf serum and/or 0.05 vol.% of Tween® 20 together with 0.02 to 0.8 M of sodium propionate

5. Process according to one of claims 2 or 3, characterised in that the dilution buffer used additionally contains 0.02 to 0.8 M of sodium propionate.

6. Process according to claim 1, characterised in that a dilution buffer is used which contains a carbonate buffer.

7. Process according to claim 6, characterised in that a dilution buffer is used which has a carbonate buffer concentration of 0.05 M and contains 5-25 vol.% of calf serum and 0.02 to 0.8 M of sodium fluoride.

8. Process according to claim 1, characterised in that a dilution buffer is used which has a carbonate buffer concentration of 0.05 M and contains 5-25 vol.% of calf serum and 0.02 to 0.8 M of sodium propionate.

9. Process according to one of claims 1 to 8, characterised in that the proteins to be determined in the body fluid are antibodies to HIV in serum or plasma.

10. Process according to one of claims 1 to 8, characterised in that the proteins to be determined in the body fluid are antibodies to the cytomegalovirus in serum or plasma.

## Revendications (Revendications pour l'(les) Etat(s) contractant(s) suivant(s): AT, BE, CH, LI, DE, FR, GB, IT, NL, SE)

1. Procédé pour éviter les réactions faussement positives lors de la détermination de protéines dans des liquides corporels, un partenaire réactionnel étant fixé à une phase solide et mis à réagir avec le partenaire à déterminer du liquide corporel, la mise en évidence s'effectuant par voie enzymatique, radioactive ou par analyse de fluorescence, caractérisé en ce qu'on ajoute à la solution contenant l'échantillon à déterminer un tampon de dilution contenant 5 à 75% d'un sérum animal et/ou 0,01 à 5% en poids ou en volume d'un tensioactif non ionique ainsi que 0,02 à 0,8M de sels inorganiques sélectionnés parmi le fluorure de sodium, l'iodure de sodium ou le bromure de sodium et/ou 0,02 à 0,8 M de propionate de sodium dans un tampon phosphate ou carbonate.

2. Procédé selon la revendication 1, caractérisé en ce qu'on utilise un tampon de dilution contenant une solution de chlorure de sodium tamponnée au phosphate avec 0,05% en volume de Tween® 20, 5 à 20% en volume de sérum de veau et 0,02 à 0,8 M de fluorure de sodium.

3. Procédé selon la revendication 1, caractérisé en ce qu'on utilise un tampon de dilution contenant 5 à 20% en volume de sérum de veau et une solution de chlorure de sodium tamponnée au phosphate et 0,02 à 0,8 M de fluorure de sodium.

4. Procédé selon la revendication 1, caractérisé en ce qu'on utilise un tampon de dilution qui contient une solution de chlorure de sodium tamponnée au phosphate, 5 à 20% en volume de sérum de veau et/ou 0,05% en volume de Tween® 20 ainsi que 0,02 à 0,8 M de propionate de sodium.

5. Procédé selon une des revendications 2 et 3, caractérisé en ce que le tampon de dilution utilisé contient additionnellement 0,02 à 0,8 M de propionate de sodium.

6. Procédé selon la revendication 1, caractérisé en ce qu'on utilise un tampon de dilution qui contient un tampon carbonate.

7. Procédé selon la revendication 6, caractérisé en ce qu'on utilise un tampon de dilution qui est un tampon carbonate 0,05 M et contient 5 à 25% en volume de sérum de veau et 0,02 à 0,8 M de fluorure de sodium.

8. Procédé selon la revendication 1, caractérisé en ce qu'on utilise un tampon de dilution qui est un tampon carbonate 0,05 M et contient 5 à 25% en volume de sérum de veau et 0,02 à 0,8 M de propionate de sodium.

9. Procédé selon une des revendications 1 à 8, caractérisé en ce que les protéines à déterminer dans le liquide corporel sont des anticorps dirigés contre le VIH dans du sérum ou du plasma.

10. Procédé selon une des revendications 1à 8, caractérisé en ce que les protéines à déterminer dans le liquide corporel sont des anticorps dirigés contre le virus cytomégalique dans du sérum ou du plasma.

11. Tampon de dilution pour éviter les réactions faussement positives lors de la détermination de protéines dans des liquides corporels, contenant 5 à 75% d'un sérum animal et/ou 0,01 à 5% en poids ou en volume d'un tensioactif non ionique ainsi que 0,02 à 0,8 M de sels inorganiques sélectionnés parmi le fluorure de sodium, l'iodure de sodium ou le bromure de sodium et/ou 0,02 à 0,8 M de propionate de sodium dans un tampon phosphate ou carbonate.

12. Tampon de dilution selon la revendication 11, caractérisé en ce qu'il contient une solution de chlorure de sodium tamponnée au phosphate avec 0,05% en volume de Tween® 20 et 5 à 20% en volume de sérum de veau ainsi que 0,02 à 0,8 M de sels inorganiques sélectionnés parmi le fluorure de sodium, l'iodure de sodium ou le bromure de sodium.

13. Tampon de dilution selon la revendication 12, caractérisé en ce qu'il contient additionnellement 0,02 à 08 M de propionate de sodium.

14. Tampon de dilution selon la revendication 11, caractérisé en ce qu'il contient un tampon carbonate, 5 à 25% en volume de sérum de veau et 0,02 à 0,8 M de sels inorganiques sélectionnés parmi le fluorure de sodium, l'iodure de sodium ou le bromure de sodium.

15. Tampon de dilution selon la revendication 14, caractérisé en ce qu'il contient additionnellement 0,02 à 0,8 M de propionate de sodium.

## Revendications (Revendications pour l'(les) Etat(s) contractant(s) suivant(s): ES)

1. Procédé pour éviter les réactions faussement positives lors de la détermination de protéines dans des liquides corporels, un partenaire réactionnel étant fixé à une phase solide et mis à réagir avec le partenaire à déterminer du liquide corporel, la mise en évidence s'effectuant par voie enzymatique, radioactive ou par analyse de fluorescence, caractérisé en ce qu'on ajoute à la solution contenant l'échantillon à déterminer un tampon de dilution contenant 5 à 75% d'un sérum animal et/ou 0,01 à 5% en poids ou en volume d'un tensioactif non ionique ainsi que 0,02 à 0,8 M de sels inorganiques sélectionnées parmi le fluorure de sodium, l'iodure de sodium ou le bromure de sodium et/ou 0,02 à 0,8M de propionate de sodium dans un tampon phosphate ou carbonate.

2. Procédé selon la revendication 1, caractérisé en ce qu'on utilise un tampon de dilution contenant une solution de chlorure de sodium tamponnée au phosphate avec 0,05% en volume de Tween® 20, 5 à 20 % en volume de sérum de veau et 0,02 à 0,8 M de fluorure de sodium.

3. Procédé selon la revendication 1, caractérisé en ce qu'on utilise un tampon de dilution contenant 5 à 20% en volume de sérum de veau et une solution de chlorure de sodium tamponnée au phosphate et 0,02 à 0,8 M de fluorure de sodium.

4. Procédé selon la revendication 1, caractérisé en ce qu'on utilise un tampon de dilution qui contient une solution de chlorure de sodium tamponnée au phosphate, 5 à 20% en volume de sérum de veau et/ou 0,05% en volume de Tween® 20 ainsi que 0,02 à 0,8 M de propionate de sodium.

5. Procédé selon une des revendications 2 et 3, caractérisé en ce que le tampon de dilution utilisé contient additionnellement 0,02 à 0,8 M de propionate de sodium.

6. Procédé selon la revendication 1, caractérisé en ce qu'on utilise un tampon de dilution qui contient un tampon carbonate.

7. Procédé selon la revendication 6, caractérisé en ce qu'on utilise un tampon de dilution qui est un tampon carbonate 0,05 M et contient 5 à 25% en volume de sérum de veau et 0,02 à 0,8 M de fluorure de sodium.

8. Procédé selon la revendication 1, caractérisé en ce qu'on utilise un tampon de dilution qui est un tampon carbonate 0,05 M et contient 5 à 25% en volume de sérum de veau et 0,02 à 0,8 M de propionate de sodium.

9. Procédé selon une des revendications 1 à 8, caractérisé en ce que les protéines à déterminer dans le liquide corporel sont des anticorps dirigés contre le VIH dans du sérum ou du plasma.

10. Procédé selon une des revendications 1 à 8, caractérisé en ce que les protéines à déterminer dans le liquide corporel sont des anticorps dirigés contre le virus cytomégalique dans du sérum ou du plasma.
